# EUROPÄISCHE PATENTSCHRIFT

(11) **EP 2 126 561 B1**
(45) Veröffentlichungstag und Bekanntmachung des Hinweises auf die Patenterteilung: **11.04.2012**
(21) Anmeldenummer: 08717202.9
(22) Anmeldetag: 28.02.2008
(51) Int. Cl.: G01N 33/34

(54) **VERFAHREN ZUR BESTIMMUNG VON HYDROPHOBEN ORGANISCHEN PARTIKELN IN EINEM PAPIERSTOFF**
METHOD FOR DETERMINING HYDROPHIC ORGANIC PARTICLES IN A PAPER FABRIC
PROCÉDÉ POUR DÉTECTER DES PARTICULES ORGANIQUES HYDROPHOBES DANS UNE PÂTE À PAPIER

(30) Priorität: 01.03.2007 EP 07103345
(43) Veröffentlichungstag der Anmeldung: 02.12.2009
(73) Patentinhaber: BASF SE, 67056 Ludwigshafen (DE)
(72) Erfinder: KAUB, Hans-Peter, 67122 Altrip (DE)
(74) Vertreter: Peatfield, Jeremy William
(86) Internationale Anmeldenummer: PCT/EP2008/052400
(87) Internationale Veröffentlichungsnummer: WO 2008/104576

(56) Entgegenhaltungen:
- WO-A-92/11534
- WO-A-2007/082376
- DOSHI MAHENDRA R ET AL: "DETECTION AND QUANTIFICATION OF STICKY CONTAMINANTS IN RECYCLED FIBER SYSTEMS" 19830101, 1. Januar 1983 (1983-01-01), Seiten 703-707, XP008094408
- OLMSTEAD J A ET AL: "FLUORESCENCE SPECTROSCOPY OF CELLULOSE, LIGNIN AND MECHANICAL PULPS: A REVIEW" JOURNAL OF PULP AND PAPER SCIENCE, LEWISTONE, NY, US, Bd. 23, Nr. 12, 1. Dezember 1997 (1997-12-01), Seiten 571-581, XP008002662 ISSN: 0826-6220

## Beschreibung

Die Erfindung betrifft ein Verfahren zur Bestimmung von hydrophoben organischen Partikeln im Filtrat eines Papierstoffes.

Bei der Herstellung von Papier ist es beispielsweise von Interesse, Störstoffteilchen im Papierstoff hinsichtlich ihrer Größenverteilung und Menge analytisch zu erfassen. Störstoffteilchen sind meistens hydrophob und klebrig. Sie stammen z. B. aus zurückgeführtem Altpapier und führen im Papierherstellungsprozess zu Ablagerungen in den Maschinen. Um den negativen Einfluss der Störstoffe auf die Papierherstellung zurückzudrängen bzw. zu beheben, dosiert man Fixiermittel zum Papierstoff. Damit erreicht man, dass die Störstoffe an die Cellulosefasern gebunden und Ablagerungen in den Maschinen weitestgehend vermieden werden. Die jeweils notwendige Fixiermittelmenge wird dann mit Hilfe einer Analyse des Papierstoffs oder des Siebwassers auf Störstoffteilchen ermittelt.

Für die Bestimmung der Größenverteilung von Störstoffteilchen in einem Papierstoff sind verschiedene Methoden bekannt. Mit herkömmlichen Untersuchungsverfahren, etwa der Röntgen-Mikroanalyse, der Infrarot-Spektralphotometrie oder der GelPermeationschromatographie, wie sie bei R. Wilken und J. Strauss, "Grundlegende Untersuchungen über klebende Verunreinigungen im wiederverwendeten Altpapier", Mitteilungen aus dem Papiertechnischen Institut der Papiertechnischen Stiftung, Band 11-12 (1984), Seite 292 ff., im Überblick beschrieben sind, kann die Art der Störstoffteilchen, also ihre chemische Zusammensetzung im Labor bestimmt werden. Es lassen sich auch qualitative Aussagen über Konzentration und Teilchengrößenverteilung treffen. Diese Verfahren haben aber allesamt den Nachteil, dass sie relativ zeit- und arbeitsaufwendig sind und damit für die unmittelbare Überwachung von Störstoffveränderungen und der Wirkung von Zusätzen auf die Bindung der Störstoffe an den Papierstoff während des Produktionszyklus nicht geeignet sind.

Ein anderes Verfahren zur Bestimmung der Teilchengrößenverteilung von Störstoffteilchen wird bei T. Kröhl, P. Lorencak, A. Gierulski, H. Eipel und D. Horn, "A new laseroptical method for counting colloidally dispersed pitch", Nordic Pulp and Paper Research Journal, Band 9 (1994), Nr.1, Seite 26 ff. beschrieben. Bei diesem Verfahren werden Störstoffteilchen mit einem Fluoreszenzfarbstoff angefärbt und durch hydrodynamische Fokussierung vereinzelt. Anschließend wird Laserlicht in die Probe mit den vereinzelten Störstoffteilchen eingestrahlt und von diesen ausgesandtes Fluoreszenzlicht aufgenommen. Aus der Intensität der Fluoreszenzsignale kann man dann auf die Teilchengrößenverteilung schließen.

Aus der WO 92/11534 ist ein Messverfahren zur Bestimmung der Anzahl und Größe von Harzteilchen in einem Papierstoff bekannt, wobei man zunächst eine Papierstoffsuspension herstellt, davon die Harzteilchen durch Filtration abtrennt, die Harzteilchen mit einem Fluoreszenzfarbstoff markiert, sie danach vereinzelt, zur Lichtemission anregt, die Lichtsignale detektiert und die Signale zur Zählung und Größenbestimmung der Harzteilchen auswertet. Als Fluoreszenzfarbstoff wird N-(n-Butyl)-4-(n-butylamino)-naphthalsäureimid verwendet.

Aus der EP-A 0 856 731 ist ein Verfahren zur Bestimmung der Größenverteilung von mindestens zwei Teilchenarten (A_{K}) von fluoreszierenden Teilchen (Tᵢ) in einer einzigen Probe bekannt, wobei die Teilchen (Tᵢ) in der Probe vereinzelt werden und Licht in die Probe entlang einer vorgegebenen Einstrahlungsrichtung eingestrahlt wird, mindestens ein Streulichtintensitätswert (S(Tᵢ)) und mindestens ein Fluoreszenzlichtintensitätswert (F(Tᵢ)) von jedem Teilchen (Tᵢ) gemessen wird,
die Teilchen (Tᵢ) aufgrund der Lage ihrer Wertepaare (S(Tᵢ),F(Tᵢ)) in einem Bereich (B_{K}) in einem dreidimensionalen Raum (R), der aus den Streulichtintensitätswerten (S(Tᵢ)), den Fluoreszenzlichtintensitätswerten (F(Tᵢ)) und der Häufigkeit der Wertepaare (S(Tᵢ),F(Tᵢ)) aufgespannt wird, jeweils einer Teilchenart (A_{K}) zugeordnet werden, wobei jeder Bereich (B_{K}) mindestens ein lokales Maximum der Häufigkeit der Wertepaare (S(Tᵢ),F(Tᵢ)) in dem Raum (R) für die Teilchenart (A_{K}) aufweist,
die relative Häufigkeit der Fluoreszenzlichtintensitätswerte (F(Tᵢ)) für jede Teilchenart (A_{K}) ermittelt wird,
die relative Teilchengrößenverteilung für jede Teilchenart (A_{K}) aus der relativen Häufigkeit der Fluoreszenzlichtintensitätswerte (F(Tᵢ)) für die entsprechende Teilchenart (A_{K}) berechnet wird,
die relativen Teilchengrößenverteilungen für die einzelnen Teilchenarten (A_{K}) mit Hilfe der Lage der Bereiche (B_{K}) in dem dreidimensionalen Raum (R), der aus den Streulichtintensitätswerten (S(Tᵢ)), den Fluoreszenzlichtintensitätswerten (F(Tᵢ)) und der Häufigkeit der Wertepaare (S(Tᵢ),F(Tᵢ)) aufgespannt wird, aufeinander normiert werden, und damit eine gemeinsame relative Teilchengrößenverteilung für die Teilchen (Tᵢ) aller Teilchenarten (A_{K}) gebildet wird.

Dieses Verfahren wird insbesondere zur Bestimmung der Teilchengrößenverteilung von hydrophoben Störstoffteilchen im Papierstoff oder im Siebwasser von Papiermaschinen benutzt und dazu verwendet, die Dosierung von Fixiermitteln zum Papierstoff zu steuern, indem ein der gemeinsamen relativen Teilchengrößenverteilung entsprechendes bzw. zugeordnetes Steuersignal erzeugt und die Dosierung der notwendigen Fixiermittelmenge aufgrund dieses Steuersignals vorgenommen wird.

Aus der WO 06/122921 ist ein Verfahren zur Bestimmung der Leimungsmittelkonzentration, der Teilchengröße und der Teilchengrößenverteilung von natürlichen und/oder synthetischen Leimungsmitteln in einem Papierstoff bekannt, wobei man eine Probe, die Leimungsmittelteilchen enthält, mit einem Fluoreszenzfarbstoff anfärbt, die Teilchen in der Probe vereinzelt, Licht einstrahlt und das von der Probe emittierte Streulicht und/oder Fluoreszenzlicht detektiert und die empfangenen Signale auswertet. Das Verfahren wird zur Bestimmung der Teilchengrößenverteilung von Reaktivleimungsmitteln im Papierstoff oder im Siebwasser von Papiermaschinen während der Papierherstellung angewendet.

Bei den oben beschriebenen Methoden werden die zu bestimmenden Teilchen immer vereinzelt. Die Einzelteilchenzählung ist jedoch aufwendig.

Der Erfindung liegt die Aufgabe zugrunde, ein anderes Messverfahren für die Bestimmung der Massekonzentration von hydrophoben Teilchen in einem Filtrat eines Papierstoffs zur Verfügung zu stellen.

Die Aufgabe wird erfindungsgemäß gelöst mit einem Verfahren zur Bestimmung der Konzentration von hydrophoben organischen Partikeln im Filtrat eines Papierstoffes, wobei man die hydrophoben organischen Partikeln mit einem Fluoreszenzfarbstoff markiert, sie anschließend zur Lichtemission anregt, das von den markierten Partikeln emittierte Licht detektiert und aus der Fluoreszenzintensität die Massenkonzentration der Partikeln bestimmt.

Hydrophobe organische Partikeln sind beispielsweise die in einem Papierstoff vorhandenen feinteiligen Störstoffe wie Stickies, White Pitch und Harz sowie Leimungsmittel. Die Störstoffe bestehen im Wesentlichen aus dispergierten hydrophoben Materialien, bei denen es sich beispielsweise um Reste von Bindemitteln von Papierstreichmassen oder um Klebstoffe handelt. Die Teilchengröße der Störstoffe beträgt beispielsweise 0,5 bis 100 µm.

Als Leimungsmittel kommen natürliche und/oder synthetische Leimungsmittel in Betracht, z. B. Reaktivleimungsmittel, Harzleim, modifizierte Harzleime oder leimend wirkende Polymerdispersionen. Bei den Leimungsmitteln handelt es sich um Verbindungen, die in Wasser dispergiert sind und beispielsweise Teilchengrößen in dem Bereich von 0,5 bis 100 µm, vorzugsweise 1 µm bis 20 µm haben.

Die wichtigsten Reaktivleimungsmittel für Papier sind Alkyldiketene (AKD) und Alkenylbernsteinsäureanhydride. Sie werden als Masseleimungsmittel bei der Herstellung von Papier, Pappe und Karton verwendet. Es handelt sich bei diesen Stoffen im Wesentlichen um C₁₄- bis C₂₂-Alkyldiketene wie Stearyldiketen, Palmityldiketen, Behenyldiketen, Oleyldiketen sowie Gemische der Diketene. Sie werden z.B. durch Emulgieren in Wasser in Gegenwart von kationischer Stärke und einem anionischen Dispergiermittel unter der Einwirkung von Scherkräften hergestellt, vgl. US 3,223,544 und US 3,130,118: Aufgrund eines Überschusses an kationischer Stärke gegenüber dem anionischen Dispergiermittel weisen die so hergestellten AKD-Disperionen eine kationische Ladung auf.

Alkyldiketene können auch zusammen mit anderen Leimungsmitteln angewendet werden. So ist beispielsweise aus der WO 94/05855 bekannt, Alkyldiketene in einer Mischung aus einer wässrigen Suspension einer aufgeschlossenen kationischen Stärke und einer feinteiligen wässrigen Polymerdispersion, die ein Leimungsmittel für Papier ist, zu dispergieren. Die resultierende Mischung wird als Leimungsmittel für Papier verwendet. Außerdem sind wässrige, anionisch eingestellte AKD-Dispersionen bekannt, die beispielsweise durch Dispergieren von AKD in Wasser in Gegenwart von anionischen Dispergiermitteln als alleinigem Stabilisator erhältlich sind, vgl. WO 00/23651.

Polymerleimungsmittel werden beispielsweise beschrieben in JP-A 58/115 196, EP-B 0 257 412 und EP-B 0 276 770. Es handelt sich dabei im Wesentlichen um wässrige Dispersionen von Copolymerisaten, die in Gegenwart von Stärke bzw. abgebauter Stärke hergestellt werden. Als Copolymerisate kommen beispielsweise Copolymere aus Styrol und/oder Acrylnitril und Acrylsäureestern in Betracht.

Alkenylbernsteinsäureanhydride werden ebenfalls bei der Herstellung von Papier und Papierprodukten als Masseleimungsmittel in der Technik verwendet. Beispiele für solche Leimungsmittel sind die isomeren 4-, 5-, 6-, 7- und 8-Hexadecenylbernsteinsäureanhydride, Decenylbernsteinsäureanhydrid, Octenylbernsteinsäureanhydrid, Dodecenylbernsteinsäureanhydrid und n-Hexadecenylbernsteinsäureanhydrid, vgl. auch C.E. Farley und R.B. Wasser, The Sizing of Paper, Second Edition, (3), Sizing With Alkenyl Succinic Anhydride, TAPPI PRESS, 1989, ISBN 0-89852-051-7.

Als natürliche Leimungsmittel kommen Harzleim sowie chemisch modifizierte Harzleime in Betracht, vgl. E. Strazdins, Chapter 1, "Chemistry and Application of Rosin Size" in W. F. Reynolds (Ed.), "The Sizing of Paper", Second Edition, Tappi Press (Atlanta, USA), 1989, Seiten 1 bis 31 (ISBN 0-89852-051-7).

Die Messungen zur integralen quantitativen Bestimmung von hydrophoben Teilchen werden in einem Fluoreszenzspektralphotometer durchgeführt. Für die Untersuchungen wurde ein Fluoreszenzspektralphotometer von Hitachi eingesetzt (Hitachi F4010). Klare Proben mit geringer Eigenextinktion werden vorzugsweise in einer 90°-Anordnung gemessen, bei der das Anregungslicht senkrecht auf die Wand der Küvette auftrifft, in der sich die zu untersuchende Probe befindet. Das emittierte Licht wird in einem Winkel von 90° zum Anregungslicht gemessen. Um trübe Lösungen und Dispersionen zu untersuchen, wählt man die Frontface-Anordnung, bei der das Anregungslicht unter einem Winkel von z.B. 45° auf die Küvette trifft und das Reflexionslicht in einem Winkel von 90° zum Anregungslicht von der Probe abgestrahlt und analysiert wird. Das Reflexionslicht wird ausgeblendet.

Die Proben, die Störstoffe und/oder ein Leimungsmittel enthalten, werden mit mindestens einem Fluoreszenzfarbstoff angefärbt. Geeignete Farbstoffe sind beispielsweise:
N-(n-Butyl)-4-(n-butylamino)-naphthalsäureimid (Fluorol 7GA),
Farbstoff der Colour Index (C.I.) Nummer 40662 (Celluflor),
Farbstoff der C.I. Nummer 45400 (Eosin B),
3,3-Ethyloxydicarbocyaninjodid,
Trinatriumsalz der 8-Hydroxy-1,3,6-pyrentrisulfonsäure,
6-Nitro-1,3,3-trimethyl-[2H]-1-benzopyran-2,2-indol (Merocyanin 540),
2-[6-(Diethylamino)-3-diethylimino-3H-xanthen-9-yl) benzoesäure (Rhodamin B).

Ein besonders bevorzugt verwendeter Fluoreszenzfarbstoff ist N-(n-Butyl)-4-(n-butylamino)-naphthalsäureimid.

Im Filtrat eines Papierstoffes findet man außerdem Cellulosefasern und gegebenenfalls anorganische Pigmente. Diese Stoffe werden von den Fluoreszenzfarbstoffen nicht angefärbt, so dass sie die Messung nicht stören. Um die Störstoffe und die Leimungsmittel mit einem Fluoreszenzfarbstoff zu markieren, benötigt man eine Einwirkungszeit von beispielsweise 2 bis 14 Minuten, vorzugsweise 2 bis 5 Minuten. Um das Analyseverfahren zu vereinfachen und um reproduzierbare Ergebnisse zu bekommen, ist es vorteilhaft, für die Färbedauer eine bestimmte Reaktionszeit festzulegen, beispielsweise 5 Minuten.

Um die zu untersuchenden Proben anzufärben, verwendet man vorzugsweise eine Lösung mindestens eines Fluoreszenzfarbstoffes in Ethanol. Die Konzentration an Fluoreszenzfarbstoff, der in Ethanol gelöst ist, beträgt beispielsweise 10 bis 40 mg/l. Die Konzentration an Störstoffen und/oder Leimungsmitteln beträgt beispielsweise 0 bis 20 mg/l.

Untersuchungen an verschiedenen Polymerteilchen (hierfür wurden handelsübliche Polymerdispersionen verwendet, die als Bindemittel für Papierstreichfarben bekannt sind, z.B. Acronal^{®} S360D und Styronal^{®} D718) ergaben einen linearen Zusammenhang zwischen der Massenkonzentration an Polymerteilchen und der gemessenen Fluoreszenzintensität. In einem Könzentrationsbereich von 0 bis 0,1 g/l an Störstoffen und/oder Leimungsmitteln kann durch die oben beschriebene Markierung mit mindestens einem Fluoreszenzfarbstoff und mit Hilfe der Bestimmung der Fluoreszenzintensität der so markierten Teilchen eine quantitative Massenanalyse von Störstoffen und/oder Leimungsmitteln durchgeführt werden. In dem angegebenen Konzentrationsbereich besteht nämlich ein linearer Zusammenhang zwischen der Massenkonzentration an Polymerteilchen und der Fluoreszenzintensität. Man erhält auf diese Weise schnell eine quantitative Analyse der Gesamtmasse an Polymerteilchen. Eine Unterscheidung zwischen verschiedenen Polymerteilchen ist jedoch nicht möglich.

Die erfindungsgemäße Meßmethode eignet sich insbesondere für schnelle Tests, um die Wirksamkeit von Prozesschemikalien zu ermitteln. Die Meßmethode findet daher Anwendung zur Bestimmung der Gesamtkonzentration an hydrophoben organischen Partikeln im Filtrat eines auf einem Sieb entwässerten Papierstoffes. Sie eignet sich insbesondere als Online-Meßmethode im Papierherstellungsprozeß.

Mit Hilfe dieser Meßmethode kann beispielsweise die Steuerung der Zugabe von Störstoffbekämpfungsmitteln und/oder Leimungsmitteln im laufenden Papierherstellungsprozess erfolgen. Ein bekanntes Störstoffbekämpfungsmittel ist z. B. hydrolysiertes Polyvinylfomamid mit einem Hydrolysegrad von beispielsweise 60 bis 95 %. Es wird dem Papierstoff vor der Blattbildung als Fixiermittel zugesetzt. Analysiert man in einem laufenden Papierherstellungsprozess das Siebwasser, so kann man darin mit Hilfe des erfindungsgemäßen Messverfahrens die Konzentration von Störstoffen ermitteln und die Dosierung von teilhydrolysiertem Polyvinylformamid zum Papierstoff so einstellen, dass das Siebwasser einen möglichst geringen Gehalt an Störstoffen aufweist. Die Störstoffe werden beispielsweise durch das zugesetzte teilhydrolysierte Polyvinylfomamid an die Cellulosefasern gebunden.

### Beispiel

Als Modellsubstanz wurde ein Sticky-System aus 8 g/l TMP-Holzschliff und 0,16 g/l Styronal® D718 (wässrige Dispersion eines Bindemittels auf Basis von Styrol und Butadien) hergestellt, indem man in einem Becherglas 500 ml dieser Mischung vorlegte und 5 ml eines hydrolysierten Polyvinylformamids mit einem Hydrolysegrad von 83 % und einem K-Wert von 61 als Fixiermittel zufügte. Der K-Wert wurde nach H. Fikentscher, Cellulose-Chemie, Band 13, 58-64 und 71-74 (1932) in 5 %iger wässriger Kochsalzlösung bei einer Temperatur von 25°C und einer Polymerkonzentration von 0,5 Gew.-% bestimmt. Nach einer Einwirkungszeit des Fixiermittels von 5 Minuten auf den Papierstoff wurde die Mischung in einem Dynamic-Drainage-Jar unter Blattbildung entwässert, wobei man die ersten 100 ml des Filtrats auffing. Zu 25 ml des Filtrats fügte man 1 ml einer ethanolischen Lösung des Fluoreszenzfarbstoffs N-(n-Butyl)-4-(n-butylamino)-naphthalsäureimid (Fluorol^{®} 7GA), durchmischte die Probe und untersuchte sie nach einer Färbezeit von 5 Minuten mit einem Fluoreszenzspektralphotometer (Hitachi F4010) in Frontface-Anordnung. Die Spaltbreite des Anregungs- und des E-missionsspaltes betrug 5 nm. Das eingestrahlte Licht hatte eine Wellenlänge von 442 nm, gemessen wurde die Fluoreszenzintensität bei 500 nm. Die Auswertung erfolgte anhand einer zuvor aufgenommenen Eichkurve für das entsprechende Sticky-System für Konzentrationen von 0 bis 0,05g/l Styronal^{®} D718. Als Sticky-Anteil im Filtrat wurde eine Konzentration von 0,01 g/l Styronal D718 ermittelt. Dieser Wert entspricht einer Retention von Styronal^{®} D718 an TMP-Holzschliff von 93,7 %.

## Patentansprüche

1. Verfahren zur Bestimmung der Konzentration von hydrophoben organischen Partikeln im Filtrat eines Papierstoffes, **dadurch gekennzeichnet, dass** man die hydrophoben organischen Partikeln mit einem Fluoreszenzfarbstoff markiert, sie anschließend zur Lichtemission anregt, das von den markierten Partikeln emittierte Licht detektiert und aus der Fluoreszenzintensität die Massenkonzentration der Partikeln bestimmt, wobei die Messungen zur integralen quantitativen Bestimmung von hydrophoben Teilchen in einem Fluoreszenzspektralphotometer durchgeführt werden.

2. Verfahren nach Anspruch 1, **dadurch gekennzeichnet, dass** man als Fluoreszenzfarbstoff N-(n-Butyl)-4-(n-butylamino)-naphthalsäureimid einsetzt.

3. Anwendung des Verfahrens nach Anspruch 1 oder 2 zur Bestimmung der Gesamtkonzentration an hydrophoben organischen Partikeln im Filtrat eines auf einem Sieb entwässerten Papierstoffes.

4. Anwendung des Verfahrens nach Anspruch 1 oder 2 als Online-Meßmethode im Papierherstellungsprozess.

5. Anwendung nach Anspruch 4, **dadurch gekennzeichnet, dass** man die Meßmethode zur Steuerung der Zugabe von Störstoffbekämpfungsmitteln und/oder Leimungsmitteln im laufenden Papierherstellungsprozess einsetzt.

## Claims

1. A method for determining the concentration of hydrophobic organic particles in the filtrate of a paper stock, wherein the hydrophobic organic particles are marked with a fluorescent dye, said particles are then excited to emit light, the light emitted by the marked particles is detected and the concentration by mass of the particles is determined from the fluorescence intensity, the measurements for the integral quantitative determination of hydrophobic particles being carried out in a fluorescence spectrophotometer.

2. The method according to claim 1, wherein the fluorescent dye used is N-(n-butyl)-4-(n-butylamino)naphthalimide.

3. The use of the method according to claim 1 or 2 for determining the total concentration of hydrophobic organic particles in the filtrate of a paper stock drained on a wire.

4. The use of the method according to claim 1 or 2 as an online measuring method in the papermaking process.

5. The use according to claim 4, wherein the measuring method is used for controlling the addition of contaminant control agents and/or sizes in the ongoing papermaking process.

## Revendications

1. Procédé pour la détermination de la concentration de particules organiques hydrophobes dans le filtrat d'une pâte à papier, **caractérisé en ce qu'**on marque avec un colorant fluorescent les particules organiques hydrophobes, puis on les excite à l'émission de lumière, on détecte la lumière émise par les particules marquées et on détermine à partir de l'intensité de fluorescence la concentration en masse des particules, les mesures étant effectuées dans un spectrophotomètre à fluorescence pour la détermination quantitative intégrale de particules hydrophobes.

2. Procédé selon la revendication 1, **caractérisé en ce qu'**on utilise comme colorant fluorescent le N-(n-butyl)-4-(n-butylamino)-naphtalimide.

3. Utilisation du procédé selon la revendication 1 ou 2 pour la détermination de la concentration totale de particules organiques hydrophobes dans le filtrat d'une pâte à papier déshydratée sur un tamis.

4. Utilisation du procédé selon la revendication 1 ou 2 en tant que méthode de mesure en ligne dans le processus de fabrication du papier.

5. Utilisation selon la revendication 4, caractisé en ce qu'on utilise la méthode de mesure pour le réglage de l'addition d'agents anti-impuretés et/ou d'agents de collage dans le processus de fabrication du papier en cours.
